# EUROPEAN PATENT APPLICATION

(11) **EP 0 716 086 A1**
(43) Date of publication of application: **12.06.1996**
(21) Application number: 94119459.9
(22) Date of filing: 09.12.1994
(51) Int. Cl.: C07D 307/52, C07C 233/54, C07F 9/38, C07F 9/30, C07C 259/06, A61K 31/34, A61K 31/16, A61K 31/66

(54) **Malonic acid based matrix metalloproteinase inhibitors**

(71) Applicant: BOEHRINGER MANNHEIM GMBH, D-68305 Mannheim (DE)
(72) Inventor: Moroder, Luis, Prof., D-82152 Martinsried (DE); Bode, Wolfram, Dr., D-82131 Gauting (DE); Grams, Frank, D-80686 München (DE); Huber, Robert, Prof. Dr., D-82110 Germering (DE)

(57) **Abstract**

There is described the use of a compound represented by the general formulae I, II or III, for the inhibition of matrix metalloproteinases (MMP), wherein X₁ is oxygen or sulfur, R₁ is OH, SH, CH₂OH, CH₂SH or NHOH, R₂ is a residue of 2 to 10 hydrocarbon backbone atoms, which binds to the amino acid 161 of HNC, said residue being saturated or unsaturated, linear or branched, and contains preferably homocyclic or heterocyclic structures, X₂ is oxygen or sulfur and binds as hydrogen acceptor on amino acid 160 of HNC, Y is a residue which binds to the S1' pocket of HNC and consists of at least 4 backbone atoms Z₁-Z₂-Z₃-Z₄-R₃, and R₃ is n-propyl, isopropyl, isobutyl or a residue with at least 4 backbone atoms, which is not larger than a tricyclic ring system. These compounds bind to MMPs in a manner different from the mode of binding of the inhibitors of the state of the art.

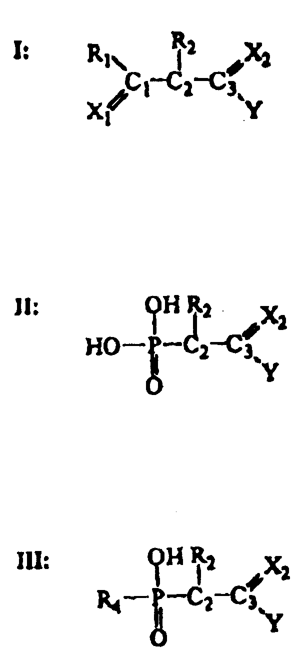

## Description

The invention comprises new matrix metalloproteinase inhibitors which are based on the structure of (pseudo)malonic acid. The invention further comprises methods for the production of the inhibitors and their use, especially in the field of therapeutics.

Matrix Metalloproteinases (MMPs, Matrixins) comprise a family of Ca-containing Zn-endopeptidases, which exhibit proteolytic activities towards most if not all of the constituents of the extracellular matrix, such as the interstitial and basement membrane collagens, fibronectin and laminin. They play a pivotal role in normal tissue remodeling and are particularily implicated in other processes such as ovulation, embryonic growth and differentiation.^{**1,2,3,4**}

At least 11 different and yet highly homologous MMP species have been characterized, including the interstitial fibroblast collagenase (MMP-1, HFC), the neutrophil collagenase (MMP-8, HNC), two gelatinases, stromelysins (such as HSL-1) and HPUMP (for a recent review, see Birkedal-Hansen et al.**²**). These proteinases share a number of structural and functional features but differ somewhat in their substrate specificity. Only HNC and HFC are capable of cleaving type I, II and III native triple-helical collagens at a single bond with the production of fragments 3/4 and 1/4 of the native chain length. This lowers the collagen melting point and makes them accessible to further attack by other matrix degrading enzymes.

All MMPs are secreted as multidomain proteolytically activatable proenzyms with a ∼ 80 residue activation peptide which in most cases is followed by the ∼ 165 residue catalytic domain terminated by a ∼ 210 residue hemopexin-like domain. The catalytic domain contains a conserved HEXXHXXGXXH zinc binding sequence characteristic for the "metzincin" superfamily**⁵** and exhibits full activity towards most small peptide substrates^{**6,7,8,20**}.

MMPs are important for normal tissue development and remodeling and have been implicated in various disease processes such as tumour growth and metastasis, rheumatoid and osteoarthritis, periodentitis, corneal ulceration, artherosclerosis and emphysema (for references see reviews^{**1,2,3,4**}). Thus, inhibitors for MMPs could be used to treat these diseases. Three endogenous protein inhibitors (TIMP-I, II ,III) which block the proteolytic activity of the MMPs in a more or less specific manner^{**10,11,12**} have been described to date. Virtually all specific synthetic collagenase inhibitors designed so far are reversible peptidyl inhibitors which interact with the active site of their target enzyme. They contain a chelating group capable of interacting with the catalytic zinc (without removing it), such as a hydroxamate, thiol, carboxylate or phosphinic group, coupled with a peptidic moiety used for binding to the substrate recognition site of the enzyme.^{**13,14,15,16**} In this way the inhibitors are targeted toward and are specific for the desired zinc enzyme.

The invention defines a new class of MMP inhibitors which bind to the MMPs in a manner completely different from the above-mentioned synthetic inhibitors.

The new inhibitors are compounds which are represented by the general formulae I, II or III, for the inhibition of matrix metalloproteinases (MMP), wherein
X₁ is oxygen or sulfur,
R₁ is OH, SH, CH₂OH, CH₂SH or NHOH,
R₂ is a residue of 2 to 10 hydrocarbon backbone atoms, which binds to the amino acid 161 of HNC, said residue being saturated or unsaturated, linear or branched, and contains preferably homocyclic or heterocyclic structures,
X₂ is oxygen or sulfur and binds as hydrogen acceptor on amino acid 160 of HNC,
Y is a residue which binds to the S1' pocket of HNC and consists of at least 4 backbone atoms Z₁-Z₂-Z₃-Z₄-R₃,
R₃ is n-propyl, isopropyl, isobutyl or a residue with at least 4 backbone atoms, which is not larger than a tricyclic ring system and
R₄ is hydrogen, alkyl or aryl, preferably isopropyl, n-butyl, benzyl.

The term "inhibition" according to the invention means a substantial reduction of collagenase activity in vitro and in vivo. The collagenase activity can be determined in vitro, for example, in an enzyme assay according to F. Grams (1993)**⁵¹**. "Substantial inhibition" means an inhibition of at least about 50% (based on collagenase activity without an inhibitor). Generally, an inhibition of more than about 80% to 90% is found.

In a preferred embodiment of the invention, the residue Y consists of a peptidic or peptidomimetic group.

The structure Z₁-Z₂-Z₃-Z₄-R₃ consists of 4 backbone atoms forming a dihedral angle of about 0° (sp2 or sp3 hybridization), wherein the distance between Z₁ and Z₄ is between 2.5 and 3.0 Å (examples see formula IV). Z₁ and Z₄ can be linked to form a cyclic structure. The preferred radicals for the cyclic substructures are peptidomimetic ring structures, such as phenylene, pyridinyl, pyrazinyl, pyrimidinyl, pyridazinyl, piperazinyl, indolinyl and morpholinyl.

Preferred residues R₃ are isopropyl, amino acid, piperidinyl, pyridinyl, furyl.

The compounds according to formulae I, II or III consist of three parts which have different structures and different properties: The chelating group (C₁R₁X₁, phosphinoyl or phosphono), the primary binding site which is referred to in the following as tail group (Y) and the secondary binding site (C₂R₂).

The chelating group of the new inhibitors interacts with the catalytic zinc (which is situated at the bottom of the active site cleft in MMPs and is penta-coordinated by tree histidines and by R₁ and X of the inhibitor) in a bidentate manner. The tail group of the inhibitors according to the invention adopts a bent conformation and inserts into the S1' pocket (subsite) and does not bind to the S2' and S3' subsites. In contrast to this, the tail group of most of the known inhibitors binds in an extended manner analogously to the active site cleft (for definition of binding sites see**³⁹**).

The reason for the difference in binding between the new inhibitors and the inhibitors of the state of the art lies in a number of essential new structural features of the inhibitors according to the invention:

### 1. The (pseudo)malonic acid basic structure.

The malonic acid structure defines three binding positions of the inhibitor. The chelating group binds via R₁ and X₁ as bidentate to the active site of zinc in the MMPs. The bidentate structure may preferably be a hydroxamate, thiol, carboxylate, phosphinoyl or a phosphono group.

The second binding site is defined by the interaction between R₂ and the amino acids 161 of the HNC. The binding derives, for example, from van der Waals or hydrophilic interaction. For an optimized binding it is preferred that R₂ is an alkyl, alkenyl, alkoxy residue with 2 to 10 backbone atoms (C, N, S) or a cyclo(hetero)alkyl or aromatic residue with 5 to 10 backbone atoms (C, N, S).

A further binding of the malonic acid basic structure to MMPs is accomplished via the oxygen or sulphur X₂ of the tail group. This oxygen or sulphur is hydrogen bonded to leucin 160 of HNC.

### 2. The tail group

The second carbonyl group (= C₃X₂) of the malonic acid basic structure is linked to the primary binding group of the inhibitors according to the invention (Y tail group, examples see formula IV). This structure comprises 4 backbone atoms forming a dihedral angle of about 0° (sp2 or sp3 hybridization). In a preferred embodiment of the invention, the turn is part of a cycle with 5 or 6 atoms. The distance between Z₁ and Z₄ is therefore preferred to be between 2.5 and 3.0 Å. At the position Z₄, there is an additional residue R₃ which is n-propyl, isopropyl, isobutyl or a residue with at least four backbone atoms, which is, however, not larger than a tricyclic ring system.

The inhibitor according to the invention binds, via Y to the S1' pocket. The pocket consists of:
1) human neutral collagenase (HNC):
   L 193, V 194, H 197, E 188, L 214, Y 216, P 217, Y 219, A 220, R 22 amino acid (numbering according to Reinemer et al. (1994)**¹⁷**.
2) HFC:
   L 181, A 182, R 214, V 215, H 218, E 219, Y 237, P 238, S 239, Y 240 amino acid (numbering according to Lovejoy et al. (1994)**⁴⁹**.
3) Stromelysin:
   L 197, V 198, H 201, E 202, L 218, Y 220, L 220, L 222, Y 223, H 224, S 225, A 226 amino acid (numbering according to Gooley et al. (1994)**⁵⁰**
Therefore, an essential feature of the compounds is that in contrast to the collagenase inhibitors according to the state of the art, the structure of the tail group is highly relevant for the inhibitory activity of the compounds according to the invention. The tail groups of the inhibitors according to the state of the art do not bind to the essential binding sites in the MMPs. In the case of the compounds according to the invention, however, the binding of the tail group Y to the S1' pocket of the MMPs constitutes the essential part of the binding between the inhibitor and collagenase and, consequently for the inhibitory activity. Thus it is essential that the tail group Y has a structure which fits well into the S1' pocket.

These requirements are fulfilled by synthetic compounds which contain a zinc chelating group which is spaced by one carbon from the substituent R₂ which constitutes an auxiliary binding site. It interacts with the surface of the protein with van der Waals and/or hydrophilic interactions. The tall group Y has to be designed for insertion into a pocket of the protein of well defined geometry and surface properties. In the upper part the environment is mainly hydrophobic, where hydrophobic interactions can be exploited, whereas the lower part contains also several hydrophilic sites, thus allowing for hydrogen bondings. Correspondingly hydrogen bond acceptors and donors are built-in in this portion of the inhibitor molecules.

Due to the short distance between the zinc binding region and X₂, in connection with the above-mentioned turn structure Y, an "L-based" structure of the inhibitor when bound to MMPs is obtained.

Most of the collagenase inhibitors according to the state of the art are based on a succinyl basic structure and show a longer distance between the zinc binding region and C₃. Therefore, R₂ binds to the S1' pocket, and there is no opportunity for the tail group to bind to this pocket. Therefore, the collagenase inhibitors according to the state of the art, in contrast to the invention, bind in a substrate-like manner and therefore show an extended backbone in the MMP bound state.

Collagenase inhibitors of this type are described, for instance, in U.S. Patent No. 4,595,700 (wherein the spacer is represented by chiral center b), U.S. Patent No. 4,599,361 (spacer represented by b or c), EP-A 0 231 081 (spacer represented by (CH₂)ₙ of formula I), EP-A 0 236 872 (spacer represented by CHR₃ of formula I), EP-A 0 276 436 (spacer represented by CH₂ of formula I), WO 90/05719 (spacer represented by the C-atom which connects a and CONHOH), EP-A 0 489 577, EP-A 0 489 579 and WO 93/14096 (spacer represented by CR₂), EP-A 0 497 192 (spacer represented by the C-atom which connects a and R₁), WO 92/16517 (spacer represented by the C-atom which connects CO₂H and CO), EP-A 0 520 573 (spacer represented by NH which connects CHCO₂H and CHR₁), WO 92/10464 (wherein the spacer is represented by one of C-atoms which connects ROCO and CCO) and WO 93/09097 (spacer represented by the C-atom which connects CONHOH and CR₂).

Further collagenase inhibitors are described in EP-A 0 320 118 and WO 92/21360. This structure differs especially by another essential feature. The molecule contains instead of C₁O, an NH group (located between CR₂ and CR₃). This NH group, in contrast to the C₁O, is an electron donor and, therefore, completely changes the properties of the molecule. From this, it is clear that this molecule cannot bind to collagenase in a fashion similar to that of the inhibitors according to the invention.

Also the collagenase inhibitors of WO 92/09563 show a completely different structure and must, therefore, bind to collagenase in a manner completely different from that of the inhibitors of the state of the art.

The above-mentioned binding properties of the inhibitor can be determined using X-ray crystallographic techniques. Such methods are described e.g. by W. Bode et al., EMBO J. 13 (1994) 1263-1269 which is incorporated herein by reference for these techniques and for the crystal structure of the catalytic domain of HNC.

### Principle features of the MMP's catalytic domain

MMPs, e.g. HNC, exhibit a spherical shape, with a shallow active-site cleft seperating a bigger "upper" N-terminal domain from a smaller "lower" C-terminal domain. The main upper domain consists of a central highly twisted five-stranded β-pleated sheet (with the β-strands ordered 2, 1, 3, 5, 4 and sheet strand 5 representing the only antiparallel strand), flanked by a double S-shaped loop and two other bridging loops on its convex side, and by two long α-helices including the active-site helix at its concave side.

Important substrate and inhibitor binding regions are the "edge" strand Leu(160) to Phe(164) of the β-sheet positioned "on top" of the active-site helix and forming the "northern" rim of the active-site cleft, and the preceeding "bulged" segment Gly(155) to Leu(160), hereafter referred to as the "bulge segment", which is part of the S-shaped double-loop. The "catalytic" zinc ion (Zn(999)) is situated at the bottom of the active-site cleft and is coordinated to the Nε2 imidazole atoms of the three histidine residues of the His(197)-Glu(198)-X-X-His(201)-X-X-Gly(204)-X-X-His(207) zinc-binding consensus sequence, and by one or two inhibitor atoms. In addition, the catalytic domain harbours a second "structural" zinc ion (Zn(998)) and two calcium ions packed against the top of the β-sheet.

The small lower domain consists of two concatenated wide loops and a C-terminal three-turn α-helix. The first of these wide right-handed loops includes a tight 1,4-turn stretching from Ala(213) to Tyr(216). This "Met-turn" represents a conserved topological element in the "metzincins"**⁵** providing a hydrophobic base for the three His residues, which ligate the catalytic zinc. The peptide chain then proceeds to the molecular surface at Pro(217) where the chain is kinked and continues in an extended strand Pro(217)-Thr(224).

The S1' pocket lies immediately to the "right" of the catalytic zinc and is formed by a long surface crevice (running perpendicular to the active-site cleft) separated from the bulk water by the initial part of this strand Pro(217)-Tyr(219) which forms its outer wall (referred to as "wall-forming segment"). The entrance to this pocket is formed by i) the bulge segment Gly(158)-Ile(159)-Leu(160) and the initial part of the edge strand Leu(160)-Ala(161) forming the "upper" side of the pocket, ii) the Tyr(219) side chain ("right" side), iii) the wall-forming segment including the Asn(218) side chain ("lower" side), and iv) the catalytic zinc together with the Glu(198) carboxylate group ("left" side). The features provide a series of polar groups for anchoring bound peptide substrates and inhibitors by hydrogen bonding (see below). The polar entrance bottleneck opens into the much more hydrophobic interior of the pocket bordered mainly by i) the side chains of Leu(160) and Val(194), ii) the Tyr(219) side chain, iii) the flat faces of the amide groups making up the wall-forming segment Pro(217)-Tyr(219), and iv) the flat side of the imidazole ring of His(197). The inner part of the pocket is filled with 4 cross hydrogen bonded "internal" water molecules in addition to the 3 to 4 solvent molecules localized in the entrance of the pocket. The bottom of the pocket is partially secluded by the long side chain of Arg(222) which is flanked by the side chains of Leu(193) and Leu(214) and extends "behind"/"below" towards the Met-turn. The terminal guanidyl group is weakly hydrogen bonded to Pro(211)O, Gly(212)O and/or Ala(213)O. Several interspersed polar groups provide anchoring points for the enclosed water molecules, one of which is in direct hydrogen bond contact with a localized bulk water molecule through an opening left between the Arg(222) side chain and the wall-forming segment.

### Binding of the inhibitors of the state of the art

In order to demonstrate the difference of binding of inhibitors of the state of the art and of inhibitors according to the invention, two model inhibitors are designed which represent the basic structure of the inhibitors according to the state of the art. These inhibitors are referred to, in the following, as MBP-AG-NH₂ and PLG-NHOH.

### Main chain interactions of inhibitors of the state of the art

The inhibitor chains of PLG-NHOH and the MBP-AG-NH₂ in complexes with HNC are bound in a more or less extended conformation. A substrate model which comprises both inhibitor conformations could be built by exchange of the zinc chelating groups by a normal peptide bond. The main chain of P3 to P3' is stabilized by four hydrogen bonds to the active site edge strand and two hydrogen bonds to the S1' pocket wall forming segment. According to this main chain conformation, the P1' Cα-Cβ bond of a MMP-bound peptide chain with L-configurated P1'-residue will point towards "back, down", allowing any more bulky (in particular aromatic) side chain to become immersed in the S1' pocket.

### The S1' subsite

The major interactions between substrates and inhibitors occur between the P1' residue and the S1' Subsite. In the MBP-AG-NH₂/collagenase complex the benzyl side chain fits into the S1' subsite which has a depth of about 9 Å and a width of 5 x 7 Å between van der Waals surfaces. The HNC hydrolyzes substrates with the relative preference at P1' of Tyr>Leu∼Met∼Ile∼Leu∼Phe>Trp>Val-Glu>Ser∼Gln∼Arg³¹⁻³³ which suggests that hydrophobic interactions are more important for binding and catalysis than polar interactions. Nevertheless, distal polar side chain groups such as the hydroxyl moiety of Tyr seem to have a beneficial effect on binding probably through hydrogen bond interactions with the enclosed water molecules. The base of the "pocket" seems to be adaptable due to the mobility of the Arg(222) side chain which can act as a flap and mantain the distinct shape of the pocket by stabilizing the wall forming segment. The S1' pocket has a narrow bottleneck, but is much more voluminous than required for any of the naturally occuring amino acids.

Interestingly, the S1' pocket of the related fibroblast collagenase differs considerably at the bottom, due to simultaneous replacements of Arg(222) of HNC by Ser, and of Leu(193) by the much longer and polar Arg in the active-site-helix forming part of the "upper" pocket wall. Similar to Arg(222) in HNC the sidechain of Arg(193) spans the bottom of the pocket in HFC which reduces the depth of the S1' pocket considerably.**¹⁸** This accounts for the much lower tolerance of HFC for Trp at P1' compared with HNC.**³¹** It might be noteworthy that no other human MMPs (except HSL-3) have Arg residues at positions 222 or 193, but contain Arg residues at positions 226 and/or 228 which could have a similar function. In this contex it is also interesting that in all MMPs but HPUMP the side chain of Val(194) is part of the S1' pocket wall. In HPUMP the Val is replaced by a Tyr. It should be mentioned, that the corresponding S1' pocket in thermolysin also is much smaller in size since it is bordered in the "back" by the active-site helix and is fully embedded in the protein matrix.

### Other Subsites

The tight fitting of the proline of PLG-NHOH into the hydrophobic cleft-like S3-subsite accounts for the beneficial effect of P3-Pro on inhibitor binding^{34,35} and to cleavage specificity,³¹⁻³³ and is in agreement with the strict occurance of Pro in any collagen substrate (see Birkedal-Hansen,1993²). Gly(II) in P1, although present in all collagen cleavage sites, does not at all utilize interactions with the cleft rims; long hydrophobic as well as polar side chains would probably improve binding. This is in agreement with cleavage activity studies³¹⁻ ³³ showing that Glu besides Ala is a more favourable P1 residue for HNC than Pro, Met, His, Tyr, Gly and Phe. The excellent property of Glu and the detrimental effect of Arg at P1 might be due to favourable and unfavourable hydrogen bond interactions with the protonated Nδ1 atom of His(162), respectively. The bad effect of Glu for cleavage by HFC might be due to a blocking effect of the nearby Asn(159) which replaces HNC's Ile(159) in HFC. Interaction of the Leu(I2) side chain allows for a considerable reduction of solvent accessible hydrophobic surfaces on both components; this side chain is, however, not voluminous enough to fill the shallow S2-subsite completely. Although Leu is apparently the optimal natural amino acid at P2,^{31,32} a tighter binding should be achievable by introducing artificial amino acids at this position.

The Ala(I2) side chain in the P2' position of MBP-AG-NH₂ does certainly not contribute well to binding, due to lack of interaction with both flanking HNC rims, mainly formed by Gly(158) and the side chain of Ile(159) on the "north", and by the side chain of Asn(218) on the "south". Indeed, most of the more powerful MMP inhibitors published so far^{30,36} contain bulky, mainly hydrophobic side chains at this position, which have been shown to assist in discriminating between different MMPs, presumably due to the replacement of Gly(158) by His (HSL-2) or Asn (HSL-1, HPUMP), and of Ile(159) by Asn (HFC), Ser (HSL-2) or Thr (HPUMP). In model peptide substrates, the replacement of Ala at this position by Phe, Trp or Leu is correlated with an increase in the hydrolysis rate for HNC and HFC, with most dramatic effects observed in case of the Trp substitution; interestingly, these increases in specificity constants primarily result from lowered K_{M}-values,^{36,38} indicating tighter interactions.

### Binding Mode of the inhibitors of the invention

The inhibitors according to the invention unexpectedly bind in a non-substrate-like geometry. This structure is a lead structure for the design of more potent collagenase inhibitors. Replacement of portions of the structure with peptidomimetic groups or non-peptide groups and filling the solvent accessible surfaces could lead to substantial improvements in inhibitor potency.

Several factors together seem to affect this strange and unforeseen binding geometry. Importantly, a favourable zinc coordination of the planar hydroxamic acid group appears to be incompatible with the proper placement of the adjacent isobutyl group in the S1' pocket. This binding geometry represents an energetic compromise since an optimal hydroxamate-zinc interaction is preferred rather than favourable embedding of the "P1'-like side chain" in the S1' pocket. An isobutyl group used as R₂ exhibits only a moderate reduction of its solvent accessible surface upon complex formation and clearly represents an appropriate point where modifications might lead to improved binding and selectivity properties. Conversely, hydroxamic acid compounds (such as BB-94³⁷) possess an additional (substituted) methylene linker between the hydroxamate group and the "P1'-like" carbon and insert their P1'-like side chains into the S1'-pocket.

The manufacture of the inhibitors according to the invention can be carried out according to the methods known in the state of the art. As starting compounds, suitable malonic acid esters are used. For the substitution of the acidic hydrogen in R₂ position with larger alkyl or aryl groups, standard base catalyzed alkylation reactions of 1,3-dicarbonyl-CH acidic compounds (or alkylation of enolates) are used.

The hydroxamates are synthesized by acylating the hydroxylamine with the malonic acid derivatives, e.g. mixed anhydride, DCC (dicyclohexylcarbodiimide) or active esters.

The oxygen of the carbonyl groups can be replaced by sulphur by using O → S exchange reagents, e.g. potassium thiocyanate^{**40,41,42**}, thiourea^{**43,44**}, 3-methylbenzothiazole-2-thione**⁴⁵** and triphenylphosphine sulfide**⁴⁶** or Lowry reagent**⁴⁷**.

In a preferred embodiment, the residue Y consists of peptidic or peptidomimetic groups. In the case of peptidic Y groups, these are coupled, e.g. via peptide coupling methods, to the malonic acid basic structure according to the methods known in the art (Houben-Weyl)**⁴⁸**. In the case of peptidomimetic groups, methods according to the state of the art are applied.

The compounds of the present invention, which specifically inhibit MMPs, are pharmacologically useful in the treatment of rheumatoid arthritis and related diseases in which collagenolytic activity is a contributing factor, such as, for example, corneal ulceration, osteoporosis, periodontitis, Paget's disease, gingivitis, tumor invasion, dystrophic epidermolysis, bullosa, systemic ulceration, epidermal ulceration, gastric ulceration, and the like. These compounds are particularly useful in the treatment of rheumatoid arthritis (primary chronic polyarthritis, PCP), systemic lupus erythematosus (SLE), juvenile rheumatoid arthritis, Sjögren's syndrome (RA + sicca syndrome), polyarteritis nodosa and related vasculitises, e.g. Wegener's granulomatosis, giant-cell arteritis, Goodpasture's syndrome, hypersensitiveness angiitis, polymyositis and dermatomyositis, progressive system sclerosis, M. Behcet, Reiter syndrome (arthritis + urethritis + conjunctivitis), mixed connective tissue disease (Sharp's syndrome), spondylitis ankylopoetica (M. Bechterew).

The compounds of the present invention may be administered by any suitable route, preferably in the form of a pharmaceutical composition adapted to such a route and in dose effective for the treatment intended. Therapeutically effective doses of the compounds of the present invention required to prevent or arrest the progress of the medical condition are readily ascertained by one of ordinary skill in the art.

Accordingly, the invention provides a class of novel pharmaceutical compositions comprising one or more compounds of the present invention, in association with one or more non-toxic pharmaceutically acceptable carriers and/or dilutions and/or adjuvants (collectively referred to herein as "carrier materials") and, if desired, other active ingredients. The compounds and compositions may, for example, be administered intravascularly, intraperitoneally, subcutaneously, intramuscularly or topically.

For all administrations, the pharmaceutical composition may in the form of, for example, a tablet, capsule, suspension or liquid. The pharmaceutical composition is preferably made in the form of a dosage unit contained in a particular amount of the active ingredient. Examples of such dosage units are tablets or capsules. A suitable daily dose for a mammal may vary widely depending on the condition of the patient and other factors. However, a dose of from about 0.1 to 300 mg/kg body weight, particularly from about 1 to 30 mg/kg body weight may be appropriate. The active ingredient may also be administered by injection.

The dose regimen for treating a disease condition with the compounds and/or compositions of this invention is selected in accordance with a variety of factors, including the type, age, weight, sex and medical conditions of the patient. Severity of the infection and the role of administration and the particular compound employed and thus may vary widely.

For therapeutic purposes, the compounds of the invention are ordinarily combined with one or more adjuvants appropriate to the indicated route of administration. If per os, the compounds may be admixed with lactose, sucrose, starch powder, cellulose esters of alkanoic acids, cellulose alkyl ester, talc, stearic acid, magnesium stearate, magnesium oxide, sodium and calcium salts of phosphoric and sulphuric acids, gelatine, acacia, sodium alginate, polyvinylpyrrolidone and/or polyvinyl alcohol, and thus tabletted or encapsulated for convenient administration. Alternatively, the compounds may be dissolved in water, polyethylene glycol, propylene glycol, ethanol, corn oil, cotton seed oil, peanut oil, sesam oil, benzyl alcohol, sodium chloride and/or various buffers. Other adjuvants and modes of administration are well and widely known in the pharmaceutical art. Appropriate dosages in any given instance, of course, depend upon the nature and severity of the condition treated, the route of administration and the species of mammal involved, including its size and any individual idiosyncracies.

Representative carriers, dilutions and adjuvants include, for example, water, lactose, gelatine starch, magnesium stearate, talc, vegetable oils, gums, polyalkylene glycols, petroleum gelly, etc. The pharmaceutical composition may be made up in a solid form, such as granules, powders or suppositories, or in liquid form, such as solutions, suspensions or emulsions. The pharmaceutical compositions may be subjected to conventional pharmaceutical operations, such as sterilization and/or may contain conventional pharmaceutical adjuvants, such as preservatives, stabilizers, wetting agents, emulsifiers, buffers, etc.

For use in the treatment of rheumatoid arthritis, the compounds of this invention can be administered by any convenient route, preferably in the form of a pharmaceutical composition adapted to such route and in a dose effective for the intended treatment. In the treatment of arthritis, administration may conveniently be by the oral route or by injection intra-articularly into the the affected joint.

As indicated, the dose administered and the treatment regimen will be dependent, for example, on the disease, the severity thereof on the patient being treated and his response to treatment and, therefore, may be widely varied.

The following examples and publications are provided to aid the understanding of the present invention, the true scope of which is set forth in the appended claims. It is understood that modifications can be made in the procedures set forth without departing from the spirit of the invention.

### Abbreviations:

HNC, MMP-8 = Human Neutrophil Collagenase, HFC, MMP-1 = Human Fibroblast Collagenase, HSL-1 = Human Stromelysin 1, HSL-2 = Human Stromelysin 2, HSL-3 = Human Stromelysin 3, HPUMP = Human PUMP, H72G = Human 72 kD Gelatinase, H92G = Human 92 kD Gelatinase, rms = root mean square, HONHiBM-AG-NH₂ = HONH-2-isobutylmalonyl-L-alanyl-glycinamide, MBP-AG-NH₂ = 2-benzyl-3-mercaptopropanoyl-L-alanylglycinamide; PLG-NHOH = L-prolyl-L-leucyl-glycin-hydroxamate.

### Example 1

### Isolation and purification of the catalytic domain of HNC

The Met(80)-Gly(242) catalytic domain of human HNC was expressed in E. coli and renatured by dialyzing the inclusion bodies which were solubilized in 6 M urea and 100 mM β-mercaptoethanol, against a buffer containing 100 mM NaCl, 5 mM CaCl₂, 0.5 mM ZnCl₂, 20 mM Tris/HCl, pH 7.5, as previously described.**²¹** The renatured enzyme was subsequently purified to apparent homogeneity as judged by SDS-PAGE by hydroxamate affinity chromatography.

### Example 2

### Synthesis of the inhibitors

The inhibitors HONH-iBM-AG-NH₂ and MBP-AG-NH₂ were synthesized according to Cushman et al. (1977)**²²**. PLG-NHOH was synthesized according to Nishino et al. (1978)**¹³**.

Inhibitors of the invention can be synthesized as described in Examples 2.1 to 2.4.

### 2.1 Isoburylmalonoyl-L-alanine-furfurylamide hydroxamate

*General:* Used solvent systems: 2E: ethyl acetate:n-butanol:acetic acid:water 5:3:1:1; 6E: ethyl acetate:n-butanol:acetic acid:water:pyridine 55:30:3:12:10; 36: cyclohexane:CHCl₃:acetic acid 45:45:10

### 1) tert-Butyloxycarbonyl-L-alanine furfurylamide (1)

To a solution of Boc-Ala-OH (10 g; 53 mmole) and N-methylmorpholine (5.8 ml; 53 mmole) in 250 ml CH₂Cl₂ isobutylchloroformate (6.3 ml; 53 mmole) was added dropwise at - 10°C under vigorous stirring. After 7 min precooled furfurylamine (7 ml; 74 mmole) was added and the reaction mixture was stirred at room temperature for 12 h. The solvent was evaporated and the residue distributed between ethyl acetate and water. The organic phase was washed with 5% KHSO₄ and 5 % NaHCO₃ and brine, dried over sodium sulfate and evaporated to dryness. The residue was recrystalized from ethyl acetate/hexane. Yield: 12.1 g (85 %); homogeneous on tlc (solvent systems: 2E, 36); mp. 107°C; [α]²⁰_{D} = -32.7°; [α]²⁰₅₄₆ₙₘ = - 38.6° (c = 1 in MeOH).

Anal. calcd. for C₁₃H₂₀N₂O₄ (268.3): C 58.19, H 7.51, N 10.44; found: C 57.83, H 7.74, N 10.22.

Using this method the following amines can be coupled with tert-butyloxycarbonyl-L-alanine: e. g. isopropylamine, butylamine, tert-butylamine, isopentylamine, hexylamine, heptylamine, octylamine, 2-octylamine, cyclohexylamine, aniline, 4-nitroaniline, 4-chloro-aniline, benzylamine, 4-chlorobenzylamine, 4-fluorobenzylamine, 2-chlorobenzylamine, 1-phenylethylamine, 2-phenylethylamine, 2-piperazin-1-yl-ethylamine, morpholine; 1-naphtylamine, fluorenyl-2-amine, dehydroabietylamine, N-(2-aminoethyl)-morpholine, (aminomethyl)pyridine, 3-(aminomethyl)pyridine, etc.

### 2) L-Alanine-furfurylamide hydrochloride (2)

Boc-Ala-Fur (2 g; 7.5 mmole) was dissolved in 1.4 M HCl/ethyl acetate. The solution was kept at room temperature for 1 h; then the solvent was evaporated and the residue reevaporated twice from toluene and finally dried on KOH pellets. Yield: 1.5 g (100%); homogeneous on tlc (solvent systems: 2E, 36); [α]²⁰_{D} = +10.3°; [α]²⁰₅₄₆ₙₘ = +12.3° (c = 1 in MeOH); FAB-MS: 169.1 [M+H]⁺. ¹H-NMR (MeOD): the spectrum is consistent with the structure.

Anal. calcd. for C₈H₁₃N₂O₂Cl (204.66): C 46.95, H 6.40, N 13.69; Found: C 46.20, H 6.62, N 13.29.

### 3) Diethyl isobutylmalonate (3)

Diethylmalonate (80g; 0.5 mole) was dissolved in a freshly prepared solution of natrium (11.5 g) in ethanol (500 ml). Then isobutylbromide (71.5 g; 0.52 mol) was added dropwise under vigorous stirring. The reaction mixture was kept under refluxing until the pH was nearly neutral (5-6 h). Insoluble material was filtered off and the reaction mixture was evaporated. The residue was distributed between water and ether and the organic phase was washed with water and dried with Na₂SO₄. The solvent was evaporated and the residue destilled under vacuum to yield the title compound as a liquid: Yield: 76 g (70%); homogeneous on tlc (solvent systems: 2E, 6E); EI-MS: 217; Anal. calcd. for C₁₁H₂₀O₄ (216.3): C 61.07 H 9.33; found: C 60.50 H 9.54.

Besides the commercially available diethyl benzylmalonate, diethyl ethoxymethylenmalonate and diethylphenylmalonate, following this procedure other malonic acid diethyl esters are prepared using e.g. 1-bromobutane, 2-bromobutane, 1-bromohexane, 2-bromohexane, 1-bromoheptane, 3-(bromomethyl)heptane, 1-bromononane, benzylbromide, bromocyclohexane, 3-bromo-1-propanol, 2-bromo-4'-methoxyacetophenone, 2-ethoxyethylbromide, 2-bromoacetophenone, N-bromomethylpthalimide.

### 4) Ethyl isobutylmalonic acid potassium salt (4)

Diethyl isobutylmalonate (1.2 g; 5.6 mmole) was dissolved in 5 ml ice-cold ethanol containing 5.6 mmole KOH. After 2 hr the solution was evaporated to small volume and the title compound was precipitated with hexane. Yield: 1.2 g (95%). ¹H-NMR (MeOD): the spectrum is consistent with the structure of the title compound.

Anal. calcd. for C₉H₁₅O₄K (226.31): C 47.77 H 6.68; found: C 45.89 H 7.93.

### 5) Isobutylmalonyl-L-alanine-furfurylamide (5)

To a chilled solution of compound **4** (0.53 g; 2.2 mmole) and in 20 ml CH₂Cl₂ oxalylchloride (0.38 ml; 4.4 mmol) was added and after 2 h at room temperature the solvent was evaporated. The residue was reevaporated from CH₂Cl₂ and finally dissolved in CH₂Cl₂ and added to a solution of **2** (0.45 g, 2.2 mmole) in CH₂Cl₂ containing triethylamine (0.61 ml; 4.4 mmole). The reaction was allowed to proceed overnight at room temperature, then the solvent was evaporated and the residue distributed between ethyl acetate and water. The organic phase was washed with 5% KHSO₄ and 5% NaHCO₃, brine. The ethyl acetate phase was dried over MgSO₄ and evaporated. The residue was dissolved in 5 ml ethanol containing 2.2 mmole KOH. After 1h the solvent was evaporated and the residue distributed between ethyl acetate and 5% KHSO_{4.} The organic phase was washed neutral, dried over Na₂SO₄ and evaporated. Yield: 0.575 g (84%); homogeneous on tlc (solvent systems: 2E, 36); FAB-MS: [M+H]⁺ = 311.2; ¹H-NMR (MeOD): consistent with the structure.

Anal. calcd. for C₁₅H₂₂N₂O₅ (310.2): C 58.04; H 7.15; N 9.03; found: C 57.77; H 7.32; N 8.89.

### 6) Isobutylmalonyl-L-alanine-furfurylamide hydroxamate (6)

Compound **5** (0.400 g; 1.3 mmole) was reacted in tetrahydrofuran with N-hydroxysuccinimide (0.148 g; 1.3 mmole) and dicyclohexylcarbodiimide (0.268 g; 1.3 mmole) in an ice bath for 5h. The dicyclohexylurea was filtered off and hydroxylamine HCl (0.181 g; 2.6 mmole) with triethylamine (0.36 ml; 2.6 mmole) in dioxan/water was added to the filtrate. The reaction was allowed to proceed overnight at room temperature. The solvent was evaporated and the residue distributed between water and ethyl acetate. The organic phase was washed with 5% KHSO₄, water and dried. The solution was concentrated and the residue precipitated with petroleum ether. Yield: 0.302 g (72%); homogeneous on tlc (solvent systems: 2E; 36). FAB-MS: [M+H]⁺ = 326.1.

Anal. calcd. for C₁₅H₂₃N₃O₅ (325.2): C 55.36, H 7.13, N 12.92; found: C 55.87, H 7.32, N 12.67.

### 2.2 2-Isobutyl-3-carbonyl-3'-(4-acetylaniline)propionic acid (7)

To a chilled solution of 4 (1.0 g; 5.3 mmole) in CH₂Cl₂ oxalylchioride (0.72 ml; 10.6 mmole) was added and after 2h at room temperature the solvent was evaporated. The residue was dissolved in CH₂Cl₂ and evaporated to remove the excess of oxalylchloride. The acid choride was dissolved in CH₂Cl₂ and added dropwise under stirring to CH₂Cl₂ containing aluminium chloride. Then a solution of acetanilide (0.72 g; 5.3 mmole) was added and the reaction mixture was kept at 20° C by cooling. The reaction mixture was treated with ice and after acidification with dilute H₂SO₄, the CH₂Cl₂ phase was separated and washed with water, dried and concentrated to small volume. The product was precipitated with petroleum ether. Yield : 0.92 g (57%); FAB-MS: [M+H]⁺ = 306.2.

The monoethyl ester (0.80 g; 2.6 mmole) was saponified in ethanol containing KOH (1 equiv.) and after 2 h the solvent was evaporated and the residue distributed between ethyl acetate and KHSO₄. The organic layer was washed with water, dried over MgSO₄ and concentrated to small volume. The title compound was isolated upon addition of petroleum ether. Yield: 0.69 g (95 %); FAB-MS: [M+H]⁺= 278.3.

Anal. calcd. for C₁₅H₁₉O₄N (277.1): C 64.95 H 6.91 N 5.05; found: C 63.67 H 7.02 N 4.99.

### 2.3 N-benzyloxycarbonyl-α-phosphonoglycyl-L-alanine furfurylamide (8)

N-(benzyloxycarbonyl)-α-phosphonoglycine trimethyl ester (1.46 g; 4.4 mmole) was completely deprotected with conc. HCl according to Balsiger et al. [(1959) J. Org. Chem. 24, 434] and the free amino function again protected with benzyloxycarbonylchloride under Schotten-Baumann conditions.

The chloridate was prepared with thionylchloride according to Balsiger et al. (1959) J.Org. Chem. 24, 434, and reacted dioxane (20 ml) with compound **2** (0.9 g; 4.4 mmole) in presence of triethylamine (4 equiv.). After 4 h at room temperature the solvent was removed and the residue distributed between ethyl acetate and KHSO₄. The organic phase was washed with water, dried over MgSO₄ and evaporated. The residue was triturated with ether/petroleum ether and filtered off. Yield 0.735 g (38%); FAB-MS: [M+H] = 439.1.

Anal. calcd. for C₁₈H₂₂N₃O₈P (439.4): C 49.21 H 5.05 N 9.56; found: C 48.95 H 5.31 N 9.43.

### 2.9 Synthon for phosphinic acid derivatives according to Formula III

The synthon can be obtained by known literature methods reviewed in Houben-Weyl, Methoden der Organischen Chemie, Vol. 12/1 and E2. Its coupling to the Y-groups, e.g. to compound **2** is achieved by classical methods of peptide synthesis and saponification of the methyl ester is performed with KOH in ethanol.

### Example 3

### Crystallization

Crystallizations were performed by hanging drop vapour diffusion at 22 °C. Droplets were made by mixing 1.8 µl of a 10 mg/ml HNC- solution in 3 mM Mes/NaOH, 100 mM NaCl, 5 mM CaCl₂, and 0.02 % NaN₃ at pH 6.0. 2 µl of an approximately 90 mM MBP-AG-NH₂ or HONHiBM-AG-NH₂ solution, and 6 µl PEG 6000 solution (10 % m/v in 0.2 M Mes/NaOH at pH 6.0). The droplets were concentrated against a reservoir buffer consisting of 0.8 M potassium phosphate buffer (with MBP-AG-NH₂) and 1.0 M (with HONHiBM-AG-NH₂), 0.02% NaN₃ at pH 6.0. Crystals of size 0.66 x 0.10 x 0.03 mm (HNC with MBP-AG-NH₂) and 0.90 x 0.12 x 0.02 mm (HNC with HONHiBM-AG-NH₂) were obtained within 3 days and harvested into 20 % (m/v) PEG 6000, 0.5 M NaCl, 0.1 M CaCl₂, 0.1 M Mes/NaOH, 0.02% NaN₃, pH 6.0 containing 10 mM of the corresponding inhibitor. The crystals belong to the orthorhombic space group P2₁2₁2₁ and exhibit lattice constants a = 33.24/33.13, b = 69.20/69.37, c = 72.33/72.31 Å, α = β = γ = 90° (HNC with MBP-AG-NH₂ / HNC with HONHiBM-AG-NH₂) and are very similar to the original Met(80)-Gly(242) collagenase crystals containing PLG-NHOH.**⁹** The asymmetric unit contains one monomer.

### Example 4

### Structure analysis

X-ray data were collected on a MAR image plate area detector (MAR Research, Hamburg) mounted on a Rigaku rotating anode X-ray generator (λ = 1.5418 Å, operated at 5.4 kW). X-ray intensities were evaluated with the MOSFILM program package,**²³** and all x-ray data were loaded with PROTEIN.**²⁴** The data collection statistics for the two complexes are given in Table 1 and compared with the data previously obtained for the HNC complex with PLG-NHOH. A 2Fo-Fc electron density map was computed using all reflection data (Table 1) and the 2.0 Å model of the Met(80)-Gly(242) form of HNC**⁹** for phasing. The nonpeptidic parts of the inhibitors were built with the program ENIGMA (a molecular graphics program provided by ICI Wilmington), and the complete inhibitor models were fitted to the electron density map using the interactive graphics program FRODO.**²⁵** The complexes were subjected to reciprocal space least squares refinement with energy constraints as implemented in X-PLOR**²⁶** using force field parameters derived by Engh and Huber.**²⁷** These refined models were compared with their improved density, rebuildt and refined to convergence. A patch residue with bond and angle energies close to zero and including the central zinc and the three surrounding HisNε2 atoms together with both hydroxamate oxygens (in the case of the HONHiBM-AG-NH2 complex) was defined for the active-site zinc. The other three metals were treated as described previously in the PLG-NHOH structure.**⁹** Water molecules previously observed in the PLG-NHOH structure were partially retained and additional waters were introduced at stereochemically reasonable positions, if appropriate density was present in maps calculated without these molecules and contoured at 1σ. In the last refinement step individual temperature factors were refined without any constrain. The final R-factor is 0.17/0.16. The final refinement statistics of the two HNC complexes is shown in Table 2 and compared with the previous data obtained with the PLG-NHOH complex.

### Example 5

### Binding of Pro-Leu-Gly-NHOH (PLG-NHOH)

The peptide chain of PLG-NHOH binds to the edge strand of HNC in a slightly twisted anti-parallel manner forming two inter-main chain hydrogen bonds with Leu(I2) and Ala(163). The N-terminal Pro(I1) fits into the hydrophobic pocket formed by the side chains of His(162), Phe(164) and Ser(151) with the Pro ring approximately parallel to the benzinering of Phe(164) and perpendicular to the His(162) imidazole group. The imino nitrogen points toward bulk water and the site of a P4 residue. The Leu(I2) side chain nestles in, but doesn't fill a shallow groove lined by His(210), Ala(206) and His(207).

The hydroxamic acid group (RCONHOH) is in the cis-configuration and is protonated due to the unambiguous involvement of the NH and OH in hydrogen bonds with protein groups. The hydroxamate hydroxyl oxygen ligates to the zinc and forms a favourable hydrogen bond (2.6 Å) with one (Oε1) of the oxygens of the Glu(198) carboxylate group. The N-H forms a hydrogenbond (3.0 Å) with the carbonyl group of the edge strand residue Ala(161).

The catalytic zinc forms a capped octahedron with both hydroxamate oxygens, His(197)Nε2, and His(207)Nε2 forming an almost tetragonal plane, and the other histidine His(201)Nε2 at the tip. The oxygen and nitrogen-zinc distances are between 1.9 and 2.2 Å, and the average angle deviation from an ideal capped octahedron is only 10° (see Table 3). The zinc ion is not exactly in the plane defined by the four nonhydrogen atoms of the hydroxamic acid, but is 0.7 Å behind it. This suggests a nonoptimal orbital interaction with the zinc presumably caused by sterical restraints in the peptide portion of the inhibitor.

Two thirds of the solvent accessible surface of the free inhibitor is removed upon complex formation in spite of the incomplete fit of the peptidyl chain. This is surprising on a first glance and might be due to the gaps between the protein surface and inhibitor which are too small to allow penetration of the solvent probe. The poor complementarity of enzyme and inhibitor could explain the relatively weak affinity of PLG-NHOH for collagenase and suggests ways for medicinal chemists to improve the structure.

### Example 6

### Binding of HS-CH2-S,R-CH(Bzl)CO-L-Ala-Gly-NH2("MBP-AG-NH2")

In the complex of MBP-AG-NH2 with the collagenase active site the sulfur atom is the fourth ligand of the catalytic zinc and together with the three imidazole nitrogens of His(197), His(201) and His(207) forms a nearly exact tetrahedron with an deviation of only 6.2° (Table 3). The refined sulfur-metal distance is 2.3 Å which is slightly longer than the average of 2.1 Å**²⁹** for Zn-S distances in proteins. The Nε2-zinc distances (between 1.9 and 2.3 Å, Table 4) are only slightly changed compared with the PLG-NHOH structure. The thiol group is presumed to coordinate the zinc in its anionic form since coordination to positively charged catalytic zinc should shift the pK to lower values.

The peptide chain of the inhibitor binds towards the "right" of the enzyme cleft in an extended geometry ("ΦI1=-179°", "ΨI1=109°", ΦI2=-89°, ΨI2=+147°, ΦI3=-93°). The inhibitor chain is almost antiparallel to bulge segment Gly(158)-Ile(159)-Leu(160), is parallel to the cross over and S1'-wall forming segment Pro(217)-Asn(218)-Tyr(219), is under the of two-rung ladders with the former (Phe(I1)O···Leu(160)N: 2.8 Å, Gly(I3)N···Gly(158)O: 3.0 Å) as well as with the latter segment (Ala(I2)N···Pro(217)O: 3.0 Å, Ala(I2)O···Tyr(219)N: 2.8 Å).

The most dominant interactions between inhibitor and enzyme are of hydrophobic manner made by the phenyl side chain and the central hydrophobic portion of the S1' pocket which is flanked by the His(197) imidazole and the Glu(198) carboxylate group (to the left), Val(194) (to the back), the phenolic side chain of Tyr(219) (to the right) and main chain segment Pro(217)-Asn(218)-Tyr(219).

The refined electron density unequivocally shows that the S-stereoisomer, corresponding to a L-amino acid analog, is preferentially bound from the inhibitor diastereomeric mixture. The Cα-Cβ bond is (according to X1 = -152°) in an essentially trans geometry with the amino group trans to Cγ.

The S1' pocket is more spacious than required to accomodate any natural amino acid side chain and could in fact bind tricyclic compounds (see below). Thus, the phenyl group of the inhibitor occupies only 1/2 to 1/3 of inner volume of S1', leaving space for three ordered solvent molecules which are at sites similar to those observed in the free enzyme. These "internal" water molecules are in partial contact with the phenyl group and are interconnected by hydrogen bonds, with themselves or hydrogen bond acceptors or donors provided by surrounding protein groups (Ala(220)N, Leu(214)O, Leu(193)O).

The side chain of Ala(I2) points away from the collagenase surface and a larger side chain would probably nestle along the shallow surface furrow running across the bulge segment between Gly(158) and Ile(159). This latter residue (Ile(159)) which is not conserved in the MMPs is presumably responsible for specificity differences among MMPs and could offer an attractive target for the design of selective inhibitors. The Gly(I3) residue is located between both cross over segments Pro(217)-Tyr(219) and Gly(158)-Leu(160). A larger side chain would collide with the enzyme and would require a rearrangement of the inhibitor chain. Additional residues could be placed on the flat molecular surface where they would find various anchoring points for polar interactions.

In summary, the thiol group and the "first residue" are involved in a large number of intimate contacts resulting in a considerable reduction of the solvent accessible surface upon binding. AIa(I2) and Gly(I3) are running along the active-site cleft, with their side chain positions extending towards the bulk solvent. The peptide binding geometry and conformation of MBP-AG-NH₂ are similar to other "primed site inhibitors" shown to bind to some other collagenases.^{**18,19**}

### Example 7

### Binding of HONHC(O)-R,S-CH(iButyl)CO-L-Ala-Gly-NH₂(HONHiBM-AG-NH₂)

The inhibitor HONHiBM-AG-NH₂ unexpectedly binds in a different manner than anticipated from its design and binding mode in thermolysin. Its hydroxamate group obviously interacts with the catalytic zinc in a favourable, bidentate manner with its two oxygen atoms and the three liganding histidines forming a trigonal-bipyramidal coordination sphere with catalytic zinc, but in contrast, its isobutyl "side chain" remains outside of the S1' pocket, presumably due to severe constraints imposed by the adjacent planar hydroxamate group. Instead, the C-terminal Ala-Gly-amide tail adopts a bent conformation and inserts into this S1' pocket, presumably in a non-optimized manner. Both the isobutyl side chain and the C-terminal peptidic tail could be replaced by other, better fitting groups. Though this inhibitor inhibits MMPs very poorly, the inhibitors according to the invention which are based on this structure are unexpectedly highly potent MMPs inhibitors. HONHiBM-AG-NH₂ is used in this example as a model substance for binding studies of the inhibitors according to the invention.

The hydroxyl oxygen, His(197)Nε2 and His(207)Nε2 form the central trigonal plane around the zinc and the carbonyl oxygen and His(201)Nε2 occupy both vertices. The average angular deviation from ideal geometry is 13.0° (see Table 3). Both the N-O and the carbonyl group of the hydroxamic acid moiety form a common plane with the catalytic zinc. As in the PLG-NHOH-complex, the hydroxamate nitrogen is close to Ala(161)O (2.9 Å) and favourably placed to form a hydrogen bond and consequently this hydroxamic acid was also modeled in its protonated form.

Due to the interaction of the hydroxamate group and the zinc "side chain" R₂ (e.g. isobutyl) is not able to insert into the S1' pocket and remains on the outer surface of the collagenase cleft exposed to solvent. The electron density accounting for this side chain is smeared out towards the periphery indicating some enhanced mobility and is loosely arranged in the crevice formed by the bulge segment and the adjacent edge strand. The S-stereoisomer fits much better, with its "side chain" arranged in a gauche⁻-conformation with the Cβ-Cγ opposite to the following carbonyl group.

In contrast to conventional "primed-site inhibitors"^{**18,19**} the L-Ala(I2)-Gly(I3)-NH₂ peptide segment binds in a bent conformation rather than an extended geometry. The carbonyl succeeding the CH(iButyl) group and the Ala(I2) carbonyl group hydrogen bond respectively with Leu(160)N of the bulge segment and Tyr(219)N of the wall-forming segment. In spite of a slight rotation of the CH(iBut)(I1)-Ala(I2) amide group out of the trans conformation, Ala(I2)NH along with the NH of Ala(I2) in MBP-AG-NH₂ is able to hydrogen bond with Pro(217)0. However, Ala(I2) has adopted a 310-helical conformation (with Φ=-78°, Ψ=-4°) and the following peptide group is oriented almost opposite to the I2-I3 amide bond of conventional "primed site inhibitors". As a consequence of this bent conformation, the amino group and the first atom of R₃ are situated in the bottleneck of the S1' pocket in van der Waals contact with Glu(198)Oε1, His(197) imidazole, and the Val(194) side chain, but with the amino group lacking any hydrogen bond acceptor.

However, the overall binding of the inhibitors of the state of the art is quite different since the side chains of R₂ (e.g. iButyl(I1)) and Z₁ to Z₃ (e.g. Ala(I2)), remain essentially exposed to water and the C-tail residue is almost completely removed from contact with it.

### Example 8

### Determination of the inhibitory effect (enzyme assay)

In order to determine the inhibition of MMPs, for example HNC, the catalytic domain (isolation and purification see example 1) is incubated with inhibitors having various concentrations. Subsequently, there is measured the initial reaction rate in the conversion of a standard substrate (F. Grams et al. (1993)^{**51**}.

The results are evaluated by plotting the reciprocal reaction rate against the concentration of the inhibitor. The inhibition constant (Kᵢ) is obtained as the negative section of the abscissis.

The synthetic collagenase substrate is a heptapeptide which is coupled, at the C-terminus, with DNP (dinitrophenol). Said DNP residue quenches by steric hindrance the fluorescence of the adjacent tryptophane of the heptapeptide. After cleavage of a tripeptide which includes the DNP group, the tryptophane fluorescence increases. The proteolytic cleavage of the substrate therefore can be measured by the fluorescence value.

The assay was performed at 25°C in a freshly prepared 50 mM Tris buffer (pH 8.0) treated with dithiozine to remove traces of heavy metals. 4 mM CaCl₂ was added and the buffer saturated with argon. Stock solutions of adamalysin II were prepared by centrifugation of the protein from an ammonium sulfate suspension and subsequent dissolution in the assay buffer. Enzyme concentrations were determined by uv measurements (ε₂₈₈ : 2.2 · 10⁴ M⁻¹ · cm⁻¹) and the stock solutions were stored in the cold. This solution was diluted 1:100 to obtain the final 16 nM assay concentration. The fluorogenic substrate DNP-ProLeu-Gly-Leu-Trp-Ala-D-Arg-NH₂ with a Kₘ of 52 µM was used at a concentration of 21.4 µM; for the Kᵢ determination a 12.8 µM concentration has also been used. Substrate fluorescence was measured at an excitation and emission wavelength of λ = 320 and 420 nm, respectively, on a spectrofluorimeter (Perkin Elmer, Model 650-40) equipped with a thermostated cell holder. Substrate hydrolysis was monitored for 10 min. immediately after adding the enzyme. All reactions were performed at least in triplicate. The Kᵢ values of the inhibitors were calculated from the intersection point of the straight lines obtained by the plots of vₒ/vᵢ vs. [concentration of inhibitor], whereas IC₅₀ values were calculated from plots of vᵢ/vₒ [concentration of inhibitor] by non-linear regression with simple robust weighting.

**Table I**

| *Statistics of data collection* | | | |
|---|---|---|---|
| | MBP-AG-NH₂ | HONHiBM-AG-NH₂ | MMP-8 with PLG-NHOH |
| Number of measurements | 20226 | 34521 | 47920 |
| Number of observations | 20050 | 33433 | 45439 |
| Number of unique reflect. (I/σ(I)>0) | 6429 | 9501 | 9740 |

| Completeness of data [%] | | | |
|---|---|---|---|
| Infinal.-smallest resolution | 92.8% (-2.40 Å) | 86.2% (-2.05 Å) | 86.3% (2.03 Å) |
| last shell (resol.[Å]) | 88.0% (2.46-2.40) | 75.7% (2.10-2.05) | 57.8% (2.07-2.03) |
| R_{Merge}¹⁾ | 12.9% | 12.1% | 11.3% |
| R_{Sym}²⁾ | 7.9% | 5.2% | 5.0% |
| Cell constants (a,b,c[Å];α,β,γ=90°) | 33.24,69.20,72.33 | 33.13,69.37,72.31 | 33.09,69.37,72.48 |

| | | | |
|---|---|---|---|
| 1) R_{Merge}=ΣhΣi(\|I(h,i)-〈I(h)〉\|)/ΣhΣiI(h,i), where I(h,i) is the intensity value of the i-th measurement of h and 〈I(h)〉 is the corresponding mean value of h for all i measurements of h; the summation is over all measurements | | | |
| 2) R_{Sym}= Σ(\|IF-〈IF〉\|)/ΣIF, where IF is the averaged value of point group related reflections and 〈IF〉 is the averaged value of a Bijvoet pair. | | | |

**Table II**

| Final refinement statistics | | | |
|---|---|---|---|
| | MBP-AG-NH₂ | MMP-8 with HONHiBM-AG-NH₂ | PLG-NHOH |
| Resolution range [Å] | 8.00-2.40 | 8.00-2.17 | 8.00-2.03 |
| Number of unique data in resol.range | 6409 | 7958 | 9600 |
| Total number of protein atoms (excl. | H) 1266 | 1266 | 1266 |
| Solvent atoms (excluding H) | 95 | 89 | 111 |

| R-factor¹⁾ | | | |
|---|---|---|---|
| 8.0-2.40/-2.17/-2.03 Å | 15.7% | 19.1% | 18.2% |
| 2.51-2.40/2.20-2.17/2.05-2.03Å | 20.4% | 24.0% | 25.1% |

| Root-mean-square deviations from target values (excluding metals and H) | | | |
|---|---|---|---|
| Bonds [Å] | 0.012 | 0.016 | 0.012 |
| Angles[°] | 1.7 | 1.9 | 1.8 |

| | | | |
|---|---|---|---|
| 1) R=(Σ\|Fo-Fc\|)/ΣFo | | | |

**Table III**

| Ligands and ligand-zinc distances and angles at the active site zinc | | | | |
|---|---|---|---|---|
| **Bond lenghts [Å]:** | | MMP8/MBP-AG-NH₂ | MMP8/HONHiBM-AG-NH₂ | MMP8 PLG-NHOH |
| Zn | Nε(197) | 1.9 | 1.9 | 2.0 |
| Zn | Nε(201) | 2.3 | 2.3 | 2.2 |
| Zn | Nε(207) | 1.9 | 2.1 | 1.9 |
| Zn | SH(Inh.) | 2.3 | | |
| SH(Inh.) | Oε1(198) | 3.0 | | |
| SH(Inh.) | Oε2(198) | 3.8 | | |
| Zn | OH(I1) | | 2.2 | 2.2 |
| Zn | O(I1) | | 2.3 | 1.9 |
| OH(I1) | Oε1(198) | | 3.2 | 2.6 |
| OH(I1) | Oε2(198) | | 3.0 | 3.4 |

| **Angles [degrees]:** | | | | |
|---|---|---|---|---|
| Nε(207)-Zn-Nε(197) | | 100.0 | 99.7 | 100.8 |
| Nε(197)-Zn-Nε(201) | | 102.4 | 102.3 | 93.7 |
| Nε(207)-Zn-Nε(201) | | 106.2 | 94.4 | 100.0 |
| Nε(207)-Zn-SH(Inh.) | | 126.1 | | |
| Nε(197)-Zn-SH(Inh.) | | 110.1 | | |
| Nε(201)-Zn-SH(Inh.) | | 109.4 | | |
| Nε(197)-Zn-OH(I1) | | | 108.1 | 84.7 |
| Nε(197)-Zn-O(I1) | | | 108.2 | 158.3 |
| Nε(201)-Zn-OH(I1) | | | 87.6 | 102.6 |
| Nε(201)-Zn-O(I1) | | | 147.1 | 102.8 |
| Nε(207)-Zn-OH(I1) | | | 151.0 | 156.4 |
| Nε(207)-Zn-O(I1) | | | 92.6 | 90.2 |
| OH(I1)-Zn-O(I1) | | | 71.5 | 78.1 |

### References:

- 1:: Woessner Jr., J. F., FASEB J. (1991) 5, 2145-2154.
- 2:: Birkedal-Hansen, H., Moore, W.G.I., Bodden, M.K., Windsor, L.J., Birkedal-Hansen, B., DeCarlo, A., Engler, J.A., Critical Rev. Oral Biol.Med. (1993) 4, 197-250.
- 3:: Murphy, G. & Docherty, A. J. P., J. Res. Cell. Mol. Biol. (1992) 7, 120-125.
- 4:: Matrisian, L. M., Trends Genet. (1990) 6, 121-125.
- 5:: Bode, W., Gomis-Rüth, F.-X., Stöcker, W., FEBS Lett. (1993) 331, 134-140.
- 6:: Sanchez-Lopez, R., Alexander, C. M., Behrendtsen, O., Breathnach, R., Werb, Z., J. Biol. Chem. (1993) 268, 7238-7247.
- 7:: Murphy, G., Allan, J. A., Willenbrock, F., Cockett, M. I., O'Connell, J. P., Docherty, A. J. P., J. Biol. Chem. (1992) 267, 9612-9618.
- 8:: Knäuper, V., Osthues, A., DeClerck, Y. A., Langley, K. E., Bläser, J., Tschesche, H., Biochem. J. (1993) 291, 847-854.
- 9:: Bode, W., Reinemer, P., Huber, R., Kleine, T., Schnierer, S., Tschesche, H., EMBO J. (1994) 13, 1263-1269.
- 10:: Docherty, A., J. P., Lyons, A., Smith, B. J., Wright, E. M., Stephens, P. E., Harris, T. J. R., (1985) Nature 318, 66-69.
- 11:: Boone, T. C., Johnson, M., J., DeClerck, Y., A., Langley, K., E., PNAS (1990) 87, 2800-2804.
- 12:: Yang, T.-T., Hawkes, S. P., PNAS (1992) 89, 10676-10680.
- 13:: Nishino, N., Powers, J.C., Biochem. (1978) 17, 2846
- 14:: Powers, J. C., Harper, J. W., (Barrett, A. J., Salvesen, G., eds.) Elvesier, Amsterdam, New York, Oxford.
- 15:: Johnson, W. H., Roberts, N. A., Borkakoti, N., J.Enz. Inhib. (1987) 2, 1-22.
- 16:: Beeley, N. R. A., Ansell, P. R. J., Docherty, A. J. P., Curr. Opin. Ther. Patents (1994) 4, 7-16.
- 17:: Reinemer, P., Grams, F., Huber, R., Kleine, T., Schnierer, S., Pieper, M., Tschesche, H., Bode, W., FEBS L. (1994) 338, 227-233.
- 18:: Stams, T., Spurlino, L., Wahl, R.C., Ho, T.F, Qoronfleth, M.W., Banks, T.M., Rubin, B., Nature Struct. Biol. (1994) 1, 119-123.
- 19:: Borkatoti, N., Winkler, F. K., Williams, D. H., D'Arcy, A., Broadhurst, M. J., Brown, P. A., Johnson, W. H., Murray, E. J., Nature Struct. Biol. (1994)1, 106-110.
- 20:: Dieckmann, O., Tschesche, H., Brazilian J. Med. Biol. Res. (1994) 27, in press.
- 21:: Schnierer, S., Kleine, T., Gote, T., Hillemann, A., Knäuper, V., Tschesche, H., Biochem. Biophys. Res. Commun. (1993) 191, 319-326.
- 22:: Cushman, D., Cheung, H. S., Sabo, E. F., Ondetti, M. A., Biochemistry (1977) 16, 5484-5491.
- 23:: Leslie, A. G. W. (1991) Daresbury Lab. Inf.Quart Prog. Crystallogr. 26 (available from the Librarian, SERC Laboratory, Daresbury, Warrington, WA4 4AD, UK).
- 24:: Steigemann, W.(1991) in: From Chemistry to Biology (Moras, D., Podjarny, A. D. and Thierry, J. C. eds) Crystallographic Computing vol. 5, pp. 115-125, Oxford University Press, Oxford, U. K.
- 25:: Jones, T. A., J. Appl. Crystallogr. (1978) 15, 23-31.
- 26:: Brünger, A. T., Karplus, M., Petsko, G. A., Acta Cryst. Sect. A (1989) 45, 50-61.
- 27:: Engh, R. A., Huber, R., Acta Cryst. Sect. A (1991) 47, 392-400.
- 28:: Kleine, T., (1994) PhD-Thesis, Universität Bielefeld.
- 29:: Chakrabarti, P., Biochemistry (1989) 28, 6081-6085.
- 30:: Beszent, B., Bird, J., Gaster, L. M., Harper, G. P., Hughes, I., Karran, E. H., Markwell, R. E., Miles-Williams, A., Smith, S. A., J. Med. Chem. (1993) 36, 4030-4039.
- 31:: Netzel-Arnett, S., Fields, G. B., Birkedal-Hansen, H., van Wart, H. E., J. Biol. Chem. (1991) 266, 6747-6755.
- 32:: Netzel-Arnett, S., Sang, Q.-X., Moore, W.G.I., Navre, M., Birkedal-Hansen, H., van Wart, H.E., Biochemistry (1993) 32, 6427-6432.
- 33:: Niedzwiecki, L., Teahan, J., Harrison, R., K., Stein, R. L., Biochemistry (1992) 31, 12618-12623.
- 34:: Moore, W. M., Spilburg, C. A., Biochemistry (1986) 25, 5189-5195.
- 35:: Odake, S., Okayama, T., Obata, M., Morikawa, T., Hattori, S., Hori, H., Nagai, Y., Chem. Pharm. Bull. (1991) 39, 1489-1494.
- 36: : Darlak, K., Miller, R. B., Stack, M. S., Spatola, A. F., Gray, R. D., J. Biol. Chem. (1990) 265, 5199-5205.
- 37:: Davies, B., Brown, P. D., East, N., Crimmin, M. J., Balkwill, F. R., Cancer Res. (1993) 53, 2087-2091.
- 38:: Holmes, M. A., Matthews, B. W., Biochemistry (1981) 20, 6912-6920.
- 39:: Schechter, J., Berger, A., Biochem. Biophys. Res. Comm. (1967) 157-162.
- 40:: Snyder, H.R., Stewart, J.M., Ziegler, J.B., J. Am. Chem. Soc. (1947) 69, 2672.
- 41:: van Tamelen, E.E., J. Am. Chem. Soc. (1951) 73, 3444.
- 42:: Price, C.C., Kirk, P.F., J. Am. Chem. Soc. (1952) 75, 2396.
- 43:: Ketcham, R., Shah, V.P., J. Org. Chem. (1963) 28, 229.
- 44:: Franks, F., Chem. Abstr. (1964) 60, 5757b.
- 45:: Calõ, V., Lopez, L., Marchese, L., Pesce, G., J. Chem. Soc. Chem. Commun. (1975) 621.
- 46:: Chan, T.H., Finkenbine, J.R., J. Am. Chem. Soc. (1972) 94, 2880.
- 47:: Lowry, O.H., Rosebrough, N.J., Faro, A.L., Randall, R.J., JBC (1951) 193, 265-275.
- 48:: Houben-Weyl, Vol. 15 I/II.
- 49:: Lovejoy, B., Cleasby, A., Hassel, A.M., Longley, K., Luther, M.A., Weigl, D., McGeehan, G., McErroy, A.B., Drewry, D., Lambert, M.H., Jordan, S.R., Science 263 (1994) 375-377
- 50:: Gooley, P.R., O'Connell, J.F., Marcy, A.I., Cuca, G.C., Salowe, S.P., Bush, B.L., Hermes, J.D., Esser, C.K., Hagmann, W.K., Springer, J.P., Johnson, B.A., Nature Struc.Biol. 1 (1994) 111-118
- 51:: F. Grams et al., FEBS 335 (1993) 76-80

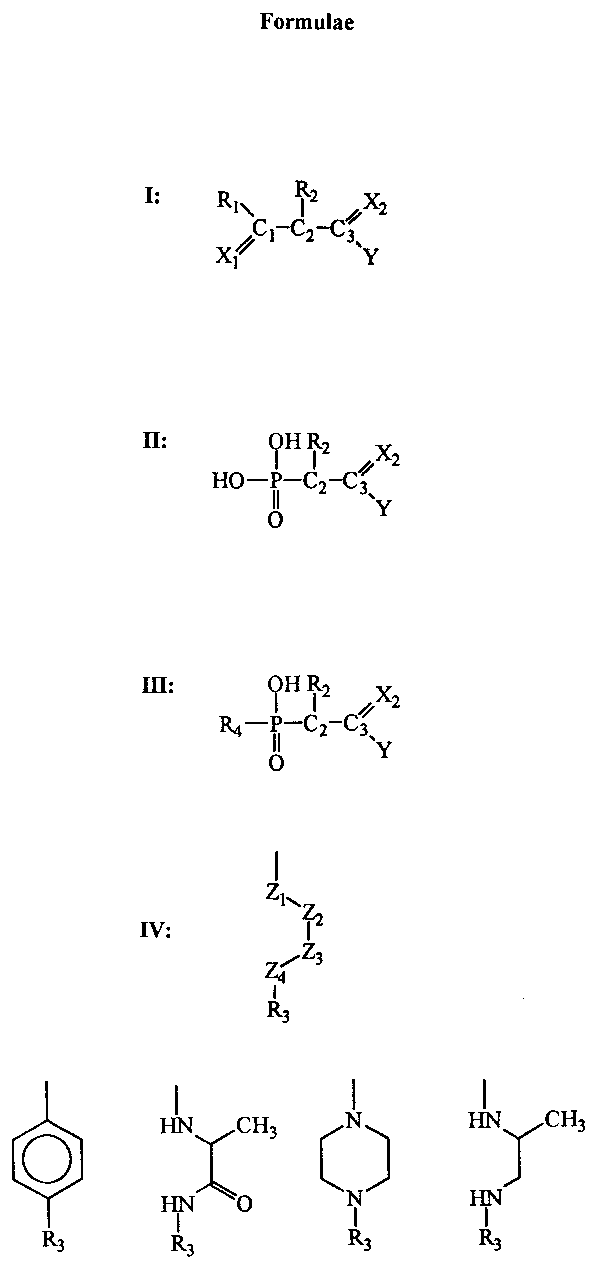

## Claims

1. A compound represented by the general formulae I, II or III, 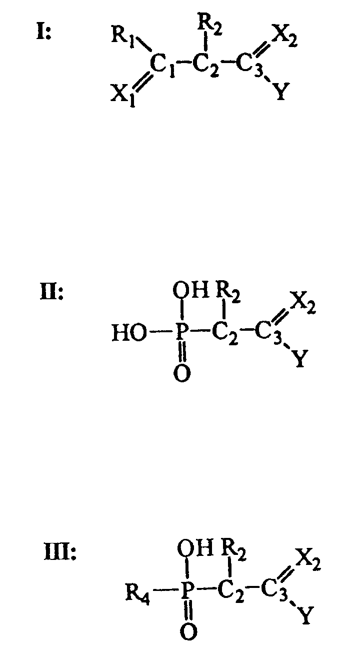 which binds and inhibits matrix metalloproteinases (MMP), wherein
X₁ is oxygen or sulfur,
R₁ is OH, SH, CH₂OH CH₂SH or NHOH,
R₂ is a residue of 2 to 10 hydrocarbon backbone atoms, which binds to the amino acid 161 of HNC, said residue being saturated or unsaturated, linear or branched, and contains preferably homocyclic or heterocyclic structures,
X₂ is oxygen or sulfur and binds as hydrogen acceptor on amino acid 160 of HNC,
Y is a residue which binds to the S1' pocket of HNC and consists of at least 4 backbone atoms Z₁-Z₂-Z₃-Z₄-R₃,
R₃ is n-propyl, isopropyl, isobutyl or a residue with at least four backbone atoms, which is not larger than a tricyclic ring system, and
R₄ is hydrogen, alkyl or aryl.

2. Compound according to claim 1, wherein R₂ contains an alkyl, alkenyl, alkoxy residue with 2 to 10 backbone atoms (C, N, S) or a cyclo(hetero)alkyl or aromatic residue with 5 to 10 backbone atoms (C, N, S).

3. Compound according to claim 1 or 2, wherein the structure Z₁-Z₂-Z₃-Z₄-R₃ consists of 4 backbone atoms forming a dihedral angle of about 0° (sp2 or sp3 hybridization), wherein the distance between Z₁ and Z₄ is between 2.5 and 3.0 Å.

4. Compound according to claims 1 to 3, wherein Z₁-Z₂-Z₃-Z₄ consists of a peptidomimetic ring structure, e.g. phenylene, pyridinyl, pyrazinyl, pyrimidinyl, pyridazinyl, piperazinyl, indolinyl and morpholinyl.

5. Compound according to claims 1 to 4, wherein Y consists of a peptidic or peptidomimetic group.

6. Compound according to claims 1 to 5, wherein Y is a residue according to formulae IV

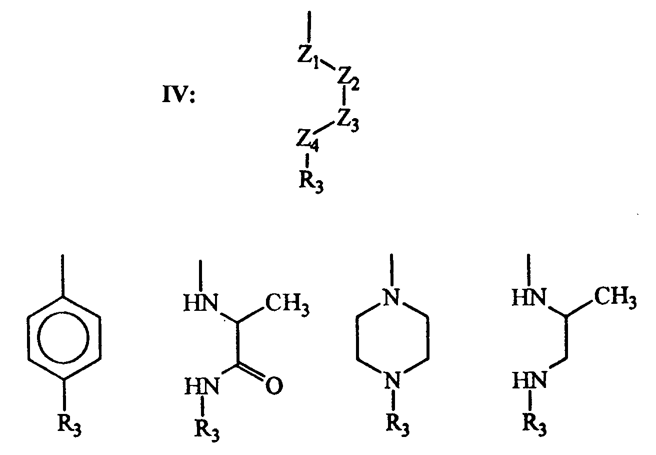

7. Compound according to claims 1 to 6, wherein R₄ is hydrogen, isopropyl, n-butyl or benzyl.

8. Use of a compound represented by the general formulae I, II or III, 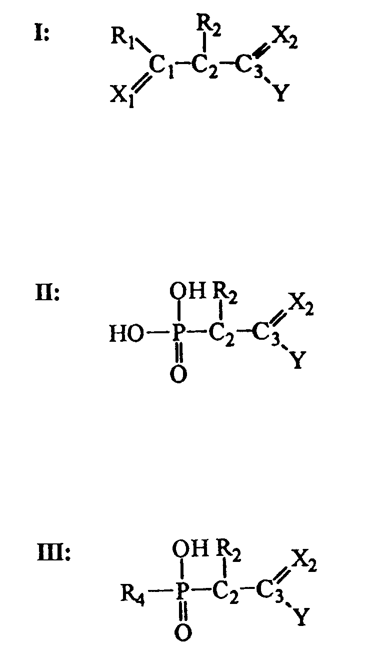 for the inhibition of matrix metalloproteinases (MMP), wherein
X₁ is oxygen or sulfur,
R₁ is OH, SH, CH₂OH, CH₂SH or NHOH,
R₂ is a residue of 2 to 10 hydrocarbon backbone atoms, which binds to the amino acid 161 of HNC, said residue being saturated or unsaturated, linear or branched, and contains preferably homocyclic or heterocyclic structures,
X₂ is oxygen or sulfur and binds as hydrogen acceptor on amino acid 160 of HNC,
Y is a residue which binds to the S1' pocket of HNC and consists of at least 4 backbone atoms Z₁-Z₂-Z₃-Z₄-R₃,
R₃ is n-propyl, isopropyl, isobutyl or a residue with at least four backbone atoms, which is not larger than a tricyclic ring system, and
R₄ is hydrogen, alkyl or aryl.

9. Use according to claim 8, wherein R₂ contains an alkyl, alkenyl, alkoxy residue with 2 to 10 backbone atoms (C, N, S) or a cyclo(hetero)alkyl or aromatic residue with 5 to 10 backbone atoms (C, N, S).

10. Use according to claim 8 or 9, wherein the structure Z₁-Z₂-Z₃-Z₄-R₃ consists of 4 backbone atoms forming a dihedral angle of about 0° (sp2 or sp3 hybridization), wherein the distance between Z₁ and Z₄ is between 2.5 and 3.0 Å.

11. Use according to claims 8 to 10, wherein Z₁-Z₂-Z₃-Z₄ consists of a peptidomimetic ring structure, e.g. phenylene, pyridinyl, pyrazinyl, pyrimidinyl, pyridazinyl, piperazinyl, indolinyl and morpholinyl.

12. Use according to claims 8 to 11, wherein Y consists of a peptidic or peptidomimetic group.

13. Use according to claims 8 to 12, wherein Y is a residue according to formulae IV

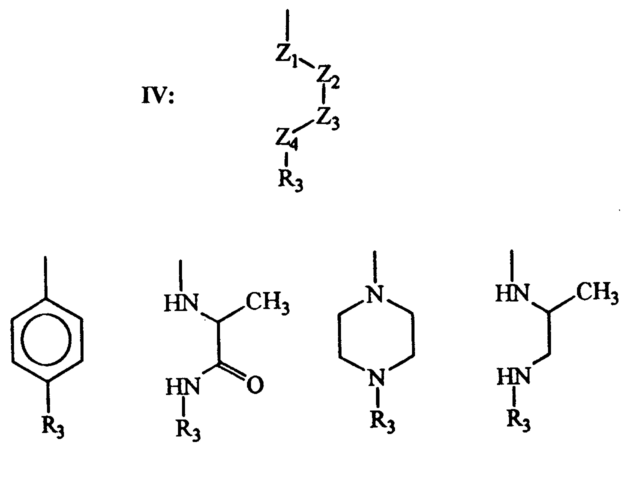

14. Use according to claims 8 to 13, wherein R₄ is hydrogen, isopropyl, n-butyl or benzyl.

15. Therapeutic composition of a compound according to claims 1 to 9.

16. Therapeutic composition according to claim 15 in association with one or more non-toxic pharmaceutically acceptable carriers and/or dilutions and/or adjuvants.

17. Use of a compound according to claims 1 to 9 for the manufacturing of a therapeutic agent for the treatment of rheumatoid arthritis and related diseases in which collagenolytic activity is a contributing factor.

18. Use according to claim 17, wherein the dose of the therapeutic agent is 0.1 to 300 mg/kg body weight.

19. Use according to claim 17 or 18, wherein the therapeutic agent is administered intravascularly, intraperitoneally, subcutaneously, intramuscularly or topically.
